(19) Europäisches Patentamt
European Patent Office
Office européen des brevets

(11) Veröffentlichungsnummer: **0 195 363**
**B1**

(12) # EUROPÄISCHE PATENTSCHRIFT

(45) Veröffentlichungstag der Patentschrift: **31.10.90**

(51) Int. Cl.[5]: **A 61 K 7/13**

(21) Anmeldenummer: **86103339.7**

(22) Anmeldetag: **12.03.86**

(54) **Haarfärbemittel.**

(30) Priorität: **20.03.85 DE 3510040**

(43) Veröffentlichungstag der Anmeldung:
**24.09.86 Patentblatt 86/39**

(45) Bekanntmachung des Hinweises auf die Patenterteilung:
**31.10.90 Patentblatt 90/44**

(84) Benannte Vertragsstaaten:
**AT BE CH DE FR GB IT LI LU NL SE**

(56) Entgegenhaltungen:
**DE-A-2 518 393**
**FR-A-2 542 193**
**US-A-2 765 341**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Rose, David, Dr.**
**Holbeinweg 7**
**D-4010 Hilden (DE)**
Erfinder: **Maak, Norbert, Dr.**
**Im Jagdfeld 41 a**
**D-4040 Neuss (DE)**
Erfinder: **Lieske, Edgar**
**Hunsrückenstrasse 40**
**D-4000 Düsseldorf (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dme Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer odere mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopryidinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferne rein gutes Aufziehvermögen auf menschlichem Haar besitzen, ohen die Kopfaut zu stark anzufärben. Die damit erzeugten Färbungen müssen eine hohe Stabilität gegen Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

Diese Anforderungen werden von den in Oxidationshaarfärbemitteln gebräuchlichen Kupplern, insbesondere von Kupplern, die p-Phenylendiamin und p-Toluylendiamin und deren Derivaten blaue Nuancen ergeben, nicht zufriedenstellend erfüllt. Besonders problematisch sind die toxikologischen Eigenschaften vieler Kuppler, z.B. vom Typ der aromatischen Amine. Aus DE—A—25 18 393 waren Tetraaminodiphenylether mit para-ständigen Aminogruppen bekannt, die als Entwicklerkomponenten in Oxidationshaarfärbemitteln geeignet sind.

Es wurde nun gefunden, daß Haarfärbemittel, enthaltend Oxidationsfärbstoffvorprodukte in einem Träger, die als Oxidationsfarbstoffvorprodukte Aminodiphenylether der Formel I

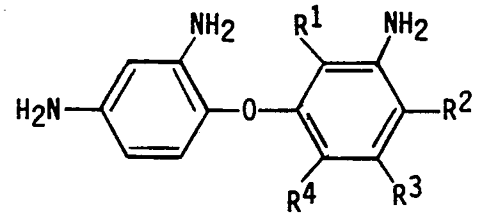

(I)

in der die Gruppen $R^1$, $R^2$, $R^3$ und $R^4$ Wasserstoff, eine Alkylgruppe mit 1—4 C-Atomen, eine Oxyalkylgruppe mit 1—4 C-Atomen oder ein Chloratom darstellen können, wobei wenigstens zwei der Gruppen $R^1$—$R^4$ Wasserstoff darstellen, oder deren Salze als Kupplersubstanzen und die Oxidationshaarfärbemitteln üblichen Entwickersubstanzen enthalten sind, die gestellten Anforderungen in hohem Maße erfüllen.

Die erfindungsgemäßen Haarfärbemittel liefern besonders intensive und brillante Haarfärbungen mit guten Echtheitseigenschaften. Bevorzugt geeignete Kupplersubstanzen der allgemeinen Formel I sind solche, bei denen die Gruppen $R^1$—$R^4$ Wasserstoff sind und ganz besonders die, bei welchen $R^1$ und/oder $R^2$ eine Methylgruppe und die übrigen Gruppen Wasserstoff darstellen oder deren Salze.

Die Kupplerverbindungen der allgemeinen Formel I sind nach literaturbekannten Verfahren herstellbar. Der 2,4-Diamino-3'-amino-4'-methyl-diphenylether und der 2,4-Diamino-3'-amino-4'-methoxy-diphenylether sind aus US—PS 2.765.341 bekannt. Allgemein können die Verbindungen der Formel I durch Veretherung eines m-Nitrophenols der allgemeinen Formel II

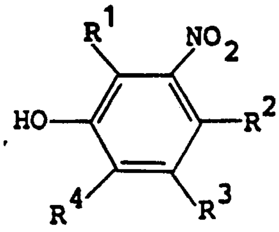

(II)

in der die Gruppen $R^1$—$R^4$ die für Formel I angegebene Bedeutung haben, mit 2,4-Dinitrofluorbenzol und katalytisch Hydrierung des erhaltenen 2,4-Dinitro-3'-nitro-diphenylethers in ansich bekannter Weise erhalten werden. Die Herstellung einiger nicht literaturbekannter 2,4-Diamino-3-amino-diphenylether der Formel I wird bei dan Beispielen beschrieben.

In die erfindungsgemäßen Haarfärbemittel können die Aminodiphenylether der Formel I in freier Form oder in Form der Salze mit anorganischen oder organischen Säuren, z.B. in Form der Hydrochloride, Sulfate, Phosphate, Acetate, Propionate, Lactate oder Citrate eingesetzt werden.

Die Verbindungen der Formel II eignen sich als Kuppler für eine Vielzahl verschiedener Entwicklersysteme, z.B. für p-Phenylendiaminderivate, p-Aminophenole und andere aromatische Amine, die wieterhin eine oder mehrere $NH_2$-Gruppen, NHR-Gruppen, $NR_2$-Gruppen, wobei R einen Alkylrest mit 1—4 oder einen Hydroxyalkylrest mit 2—4 Kohlenstoffatomen darstellt, enthalten, ferner Diaminopyridinderivate und 2.4.5.6-Tetraaminopyrimidin und dessen Derivate wie 4.5-Diamino-2.6-bis-methylaminopyrimidin, 2.5-Diamino-4-diethylamino-6-methylaminopyrimidin, 2.4.5-Triamino-6-anilinopyrimidin, 2.4.5-Triamino-6-morpholino-pyrimidin, 2.4.5-Triamino-6-(2-hydroxyethyl)-aminopyrimidin.

Besonders intensive und reine blaue Farbnuancen werden bei Verwendung der neuen Kupplersubstanzen in Oxidatinsfärbemitteln zusammen mit Entwicklersubstanzen von Typ der aromatischen Diamine, insbesondere der p-Phenylendiaminderivate erhalten. Solche blauen Nuancen sind für die Erzeugung von Braun- und Schwarztönen durch Kombination mit gelben und roten Nuancen sowie zur Kompensation des Verrötungseffektes in Blondiermitteln von hohen anwendungstechnischem Wert.

Solche bevorzugten Entwicklersubstanzen sind z.B. p-Phenylendiamin, p-Toluylendiamin, N-Methyl-p-phenylendiamin, N,N-Dimethyl-p-phenylendiamin, N-Hydroxyethyl-p-phenylendiamin, N,N-Bis-(2-hydroxyethyl)-p-phenylendiamin, N-Ethyl-N-(2-hydroxyethyl)-p-phenylendiamin, N,N-Diethyl-2-methyl-p-phenylendiamin, 2-Chlor-p-phenylendiamin, 2,6-Dichlor-p-phenylendiamin, 2-Chlor-6-methyl-p-phenylendiamin, 2-Methoxy-p-phenylendiamin, 2,5-Diaminoanisol, 6-Methoxy-3-methyl-p-phenylendiamin, N-(2-Methoxyethyl)-p-phenylendiamin, N-(2-Hydroxypropyl)-p-phenylendiamin, N-Butyl-N-sulfobutyl-p-phenylendiamin, N-(p-Aminophenyl)-N',N'-bis-(β-hydroxyethyl)-1,3-diaminopropan oder deren Salze mit anorganischen oder organischen Säuren.

Die erfindungsgemäßen Haarfärbemittel können neben den Aminodiphenylethern der allgemeinen Formel I auch andere bekannte Kupplersubstanzen enthaltne, die zur Modifizierung der Farbnuancen und zur Erzeugung natürlicher Farbtöne erforderlich sind. Solche üblichen Kupplerverbindungen sind z.B. andere m-Phenylendiamine, z.B. 2.4-Diaminophenyl-2-hydroxyethylether, 2.4-Diaminoanisol oder Phenole, Resorcine, m-Aminophenole, Naphthole oder Pyrazolone.

Gegenebenfalls können auch direktziehende Farbstoffe zusätzlich zur weiteren Modifizerung der Farbnuancen eingesetzt werden. Solche direktziehenden Farbstoffe sind z.B. Nitrophenylendiamine, Nitroaminophenole, Anthrachinonfarbstoffe oder Indophenole.

Zu den erfindungsgemäßen Haarfärbemitteln werde die 2.4-Amino-3'-amino-diphenylether der allgemeinen Formel I und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erweisen hat, so ist ein gewisser überschuß einzelner Oxidationsfarbstoffvorprodukte nicht nachteilig, so daß Entwicklersubstanzen und Kupplersubstanzen in einem Molverhältnis von 1:0,5 bis 1:2 enthalten sein können.

Es ist nicht erforderlich, daß die Aminodiphenylether der allgemeinen Formel I sowie die sonst in den Haarfärbemitteln vorhandenen Oxidationsfarbstoffvorprodukte oder direkt ziehenden Farbstoffe einheitlich chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäurester und Fettsäurepartialglyceride, Fettsäurealkanolamide sowie Verdickungsmittel wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesezt, z.B. werden Emulgiermittel in Konzentrationen von 0,5—30 Gew.-% und Verdickungsmittel in Konzentrationen von 0,1—25 Gew.-% des gesamten Färbemittels eingesetzt. Die Oxidationsfarbstoffvorprodukte werden in Mengen von 0,2—5 Gew.-%, vorzugsweise 1—3 Gew.-% des gesamten Färbemittels in den Träger eingemischt. Der Gehalt an Aminodiphenylethern der Formel I kann in den erfindungsgemäßen Haarfärbemitteln etwa 0,05—10 Millmil pro 100 g des Haarfärbemittels betragen.

Die Anwendung der erfindungsgemäßen Haarfärbemittel kann, unabhängig von der Art der kosmetischen Zubereitung, z.B. als Cremme, Gel oder Shampoo im schwach sauren, neutralen oder

alkalischen Milieu erfolgen. Bevorzugt ist die Anwendung der Haarfärbemittel in einem pH-Bereich von 8—10. Die Anwendungstemperaturen können in einem Bereich zwischen 15°C und 40°C liegen.

Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo verwendet wurde.

Die nachfolgenden Beispiele sollen den Erfindungsgegenstand näher erläutern ohen ihn jedoch hierauf zu beschränken.

## Beispiele

1. Herstellung von Aminodiphenylethern der Formel I

1.1 *2.4-Diamino-3'-aminodiphenylether-trihydrochlorid*

1. Stufe

Zu einer Mischung aus 13,9 g (0,1 Mol) m-Nitrophenol und 10 ml (0,07 Mol) Triethylamin wurde eine Lösung von 18,6 g (0,1 Mol), 2.4-Dinitrofluorbenzol in 100 ml Aceton bei 25°C zugetropft. Nach 40 Minuten Siden unter Rückfluß wurde die Lösung zur Trockne eingeengt. Der Rückstand wurde mit 200 ml einer 5 Gew.-%igen wäßrigen Salzsäure versetzt und filtriert. Nach Trocknung bei 50°C im Vakuum ergab sich eine Ausbeute von 30,2 g 2.4-Dinitro-3'-nitrodiphenylether (99% d.Th.) in Form gelber Kristalle mit einem Schmelzpunkt von 123°C.

2. Stufe

10 g 2.4-Dinitro-3'-nitrophenylether wurden in 300 ml Ethanol gelöst, mit 0,5 g einer mit 5 Gew.-% Palladium beladenen Aktivkohle versetzt und in Wasserstoffatmosphäre hydriert. Nach beendeter Wasserstoffaufnahme wurde der Katalysator abgetrennt und die Lösung mit verdünnter Salzsäure angesäuert und zur Trockene eingeengt. Es wurden violette Kristalle mit einem Schmelzpunkt von 125°C (unter Zersetzung) erhalten.

1.2 *2.4-Diamino-2'-methyl-3'-aminodiphenylether-trihydrochlorid*

Nach dem unter 1.1 beschriebenen Verfahren wurde aus 2-Methyl-3-nitrophenol der 2.4-Dinitro-2'-methyl-3'-nitrodiphenylether in Form gelber Kristalle mit einem Schmelzpunkt von 146—149°C erhalten. Daraus wurde durch katalytische Hydrierung das 2.4-Diamino-2'-methyl-3'-aminodiphenylether-trihydrochlorid in Form roter Kristalle mit einem Schmelzpunkt von 240°C (unter Zersetzung) erhalten.

1.3 *2.4-Diamino-3'-amino-4'-methyldiphenylether-trihydrochlorid*

Nach dem unter 1.1 beschriebenen Verfahren wurde aus 4-Methyl-3-nitrophenol der 2.4-Dinitro-3'-nitro-4'-methyldiphenylether in Form gelber Kristalle mit einem Schmelzpunkt von 114—117°C erhalten. Daraus wurde durch katalytische Hydrierung das 2.4-Diamino-3'-amino-4'-methyldiphenylether-trihydrochlorid in Form roter Kristalle mit einem Schmelzpunkt von 215°C (unter Zersetzung) erhalten.

1.4 *2.4-Diamino-6'-methoxy-3'-aminodiphenylether-trihydrochlorid*

Nach dem unter 1.1 beschriebenen Verfahren wurde aus 3-Nitro-6-methoxyphenol der 2.4-Dinitro-3'-nitro-6'-methoxydiphenylether in Form gelber Kristalle mit einem Schmelzpunkt von 156—158°C erhalten. Daraus wurde durch katalytische Hydrierung das 2.4-Diamino-6'-methoxy-3'-aminodiphenylether-trihydrochlorid in Form roter Kristalle mit einem Schmelzpunkt von 202°C (unter Zersetzung) erhalten.

1.5 *2.4-Diamino-2',4'-dichlor-3'-aminodiphenylether-trihydrochlorid(monohydrat)*

Nach dem unter 1.1 beschriebenen Verfahren wurde aus 2,4-Dichlor-3-nitrophenol der 2.4-Dinitro-2',4'-dichloro-3'-nitrodiphenylether in Form von gelben Kristallen mit einem Schmelzpunkt von 167°C erhalten.

7,5 g (0,02 Mol) des 2.4-Dinitro-2',4'-dichlor-3'-nitrodiphenylethers und 67,7 g (0,3 Mol) $SnCl_2$-$2H_2O$ in 80 ml Ethanol wurden 3 Stunden auf 80°C unter Rückflußkühlung erhitzt. Nach dem Abkühlen auf 20°C wurde der Ansatz auf 200 ml Eiswasser gegossen und durch Zugabe von $NaHCO_3$-Lösung auf einen pH-Wert von ca. 7 eingestellt und das Reaktionsgemisch freimal mit je 200 ml Ethylacetat ausgeschüttelt. Die organische Phase wurde über $Na_2SO_4$ getrocknet und dann zur Trockne eingeengt. Der Rückstand wurde mit verdünter Salzsäure versetzt und erneut zur Trockene eingeengt. Das 2.4-Diamino-2',4'-dichlor-3'-aminodiphenylether-trihydrochlorid(monohydrat) wurde in Form brauner Kristalle mit einem Schmelzpunkt von 170° (unter Zersetzung) erhalten.

2. Anwendungstechnische Prüfungen

Es wurden erfindungsgemäße Haarfärbemittel in Form einer Haarfärbe-Cremeemulsion der folgenden Zusammensetzung hergestellt.

| | |
|---|---|
| Fettalkohol $C_{12-14}$ | 10 g |
| Fettalkohol $C_{12-14}$ + 2EO-sulfat, Na-Salz 28 %ig | 25 g |
| Wasser | 60 g |
| Kupplersubstanz | 0,0075 Mol |
| Entwicklersubstanz | 0,0075 Mol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90% ergrauten, aber nicht besonders vorbehandelten Menschenhaars aufgregen und dort 30 Minuten bei 27°C belassen. Nach Beedigung des Färbeprozesses wurde das Haar gespült, mit einem üblichen Haarwaschmittel ausgewaschen und anschließend getrocknet.

Als Kupplersubstanzen wurden die folgenden Verbindungen eingesetzt:

K1: 2.4-Diamino-3'-aminodiphenylether (·3HCl) (nach Beispiel 1.1)
K2: 2.4-Diamino-2'-methyl-3'-aminodiphenylether (·3HCl) (nach Beispiel 1.2)
K3: 2.4-Diamino-3'-amino-4'-methyldiphenylether (·3HCl) (nach Beispiel 1.3)
K4: 2.4-Diamino-6'-methoxy-3'-aminodiphenylether (·3HCl) (nach Beispiel 1.4)
K5: 2.4-Diamino-2',4'-dichlor-3'-aminodiphenylether (·3HCl·$H_2O$) (nach Beispiel 1.5)

Als Entwicklersubstanzen wurden die folgenden Verbindungen eingesetzt:

E1: p-Phenylendiamin
E2: p-Toluylendiamin
E3: N-Methyl-p-phenylendiamin
E4: 2-Chlor-p-phenylendiamin
E5: N,N-Diethyl-p-phenylendiamin
E6: N-(2-Hydroxyethyl)-p-phenyldiamin
E7: N-(2-Hydroxypropyl)-phenylendiamin
E8: N,N-Bis-(2-hydroxyethyl)-p-phenylendiamin
E9: N-(2-Methoxyethyl)-p-phenylendiamin
E10: 2,5-Diaminoanisol
E11: N-(p-Aminophenyl)-N',N',-bis-(2-hydroxyethyl)-1.3-diaminopropan
E12: 2,5-Diaminopyridin
E13: p-Aminophenol
E14: 2,4,5,6-Tetraaminopyrimidin
E15: 2-Methylamino-4,5,6-triaminopyrimidin
E16: 2-Dimethylamino-4,5,6-triaminopyrimidin
E17: 2-Piperidino-4,5,6-triaminopyrmidin.

Die mit diesen Oxidationsfarbstoffvorprodukten erhaltenen Haaranfärbungen sind der folgenden Tabelle zu entnehmen:

Tabelle

| Anwendungs-beispiel | Kuppler K | Entwickler E | Nuance der gefärbten Haarsträhnen |
|---|---|---|---|
| 2.1 | K 1 | E 1 | dunkelviolett |
| 2.2 | K 1 | E 2 | dunkelblau |
| 2.3 | K 1 | E 7 | tintenblau |
| 2.4 | K 1 | E 8 | nordischblau |
| 2.5 | K 1 | E 11 | blaugrau |
| 2.6 | K 1 | E 14 | dunkelgrün |
| 2.7 | K 1 | E 15 | grün |
| 2.8 | K 2 | E 1 | dunkelviolett |
| 2.9 | K 2 | E 2 | dunkelblau |
| 2.10 | K 2 | E 3 | schwarzblau |
| 2.11 | K 2 | E 9 | schwarzblau |
| 2.12 | K 2 | E 10 | tintenblau |
| 2.13 | K 2 | E 13 | violettbraun |
| 2.14 | K 3 | E 2 | blauschwarz |
| 2.15 | K 3 | E 10 | dunkelblau |
| 2.16 | K 3 | E 12 | rotbraun |
| 2.17 | K 3 | E 13 | violettbraun |
| 2.18 | K 3 | E 16 | dunkeltürkis |
| 2.19 | K 3 | E 17 | dunkeltürkis |
| 2.20 | K 4 | E 2 | blauschwarz |
| 2.21 | K 4 | E 6 | schwarzblau |
| 2.22 | K 5 | E 4 | grauviolett |
| 2.23 | K 5 | E 5 | dunkelblau |
| 2.24 | K 5 | E 15 | mattgrün |
| 2.25 | K 5 | E 16 | mattgrün |
| 2.26 | K 5 | E 17 | mattgrün |

## Patentansprüche

1. Haarfärbemittel, enthaltend Oxidationsfärbstoffvorprodukte in einem Träger, dadurch gekennzeichnet, daß als Oxidationsfarbstoffvorprodukte Aminodiphenylether der Formel I

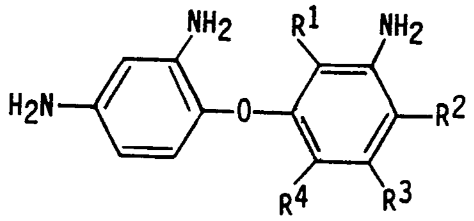

(I)

in der die Gruppen $R^1$, $R^2$, $R^3$ und $R^4$ Wasserstoff, eine Alkylgruppe mit 1—4 C-Atomen, eine Oxyalkylgruppe mit 1—4 C-Atomen oder ein Chloratom darstellen können, mit der Maßgabe, daß wenigstens zwei der Gruppen $R^1$—$R^4$ Wasserstoff darstellen, oder deren Salze als Kupplersubstanzen und die Oxidationshaarfärbemitteln üblichen Entwicklersubstanzen enthalten sind.

2. Haarfärbemittel nach Anspruch 1, dadurch gekennzeichnet, daß als Kupplersubstanzen Aminodiphenylether der allgemeinen Formel I nach Anspruch 1, in der $R^1$ und/oder $R^2$ eine Methylgruppe und die übrigen Gruppen Wasserstoff darstellten oder deren Salze enthalten sind.

3. Haarfärbemittel nach Anspruch 1 oder 2, dadurch gekennzeichnet, daß als Entwicklersubstanzen aromatische Diamine enthalten sind.

4. Haarfärbemittel nach Anspruch 1—3, dadurch gekennzeichnet, daß das zusätzlich weitere, bekannte Kupplersubstanzen enthalten sind, daß Entwicklersubstanzen und Kupplersubstanzen im Molverhältnis 1:0,5 bis 1:2 enthalten sind, daß der Gehalt an Oxidationsfarbstoffvorprodukten 0,2—5,0 Gew.-% des Haarfärbemittels und der Gehalt an Aminodiphenylethern der Formel I 0,05—10 Millimol pro 100 g des Haarfärbemittels beträgt.

## Revendications

1. Teintures pour cheveux comportant des précurseurs de colorants d'oxydation dans un support, caractérisées en ce que sont contenus en tant que précurseurs de colorants d'oxydation des aminodiphényléthers de formule I

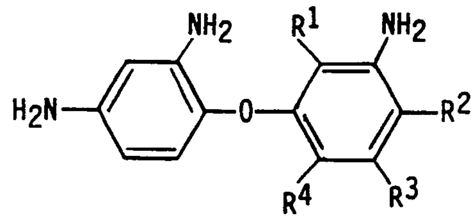

(I)

dans laquelle les groupements $R^1$, $R^2$, $R^3$ et $R^4$ peuvent représenter l'hydrogène, un groupement alkyle comportant 1 à 4 atomes de C, un groupement oxyalkyle comportant 1 à 4 atomes de C ou un atome de chlore, à la condition qu'au moins deux des groupements $R^1$ à $R^4$ correspondent à l'hydrogène, ou leurs sels à titre de substances de couplage ainsi que les substances de développement usuelles dans les teintures de cheveux par oxydation.

2. Teintures pour cheveux selon la revendication 1, caractérisées en ce qu'elles contiennent comme substances de couplage des aminodiphényléthers de la formule générale I, selon la revendication I, dans laquelle $R^1$ et/ou $R^2$ représentent un groupement méthyle et les autres groupements de l'hydrogène, ou leurs sels.

3. Teintures pour cheveux selon la revendication 1 ou 2, caractérisées ence qu'elles contiennent comme substances de développement des diamines aromatiques.

4. Teintures pour cheveux selon la revendication 1 ou 2, caractérisées en ce qu'elles contiennent en outre d'autres substances de couplage connues, en ce que les substances de développement et les substances de couplage sont présentes dans un rapport molaire compris dans l'intervalle de 1:05 à 1:2, en ce que le contenu en précurseurs de colorants d'oxydation atteint 0,2 à 5,0% en poids de la teinture, et en ce que la concentration en aminodiphényléthers de la formule I se situe dans la plage s'étendant de 0,05 à 10 millimoles par 100 g de teinture pour cheveux.

**Claims**

1. Hair dyes containing oxidation dye precursors in a support, characterized in that the oxidation dye precursors are aminodiphenylethers corresponding to formula I

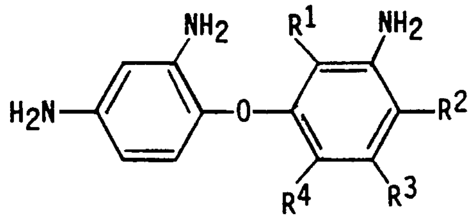

(I)

in which the substituents $R^1$, $R^2$, $R^3$ and $R^4$ may be hydrogen, a $C_{1-4}$ alkyl group, a $C_{1-4}$ hydroxyalkyl group or a chlorine atom, with the proviso that at least two of the substituents $R^1$ to $R^4$ are hydrogen, or salts thereof as couplers and the developers typically present in oxidation hair dyes.

2. Hair dyes as claimed in claim 1, characterized in that the couplers are aminodiphenylethers corresponding to general formula I in claim 1, in which $R^1$ and/or $R^2$ represent a methyl group and the other substituents are hydrogen, or salts thereof.

3. Hair dyes as claimed in claim 1 or 2, characterized in that the developers are aromatic diamines.

4. Hair dyes as claimed in claims 1 to 3, characterized in that other known couplers are additionally present, in that the developers and couplers are present in a molar ratio of 1:0.5 to 1:2 and in that the content of oxidation dyes precursors is from 0.2 to 5.0% by weight, based on the hair dye, and the content of aminodiphenylethers of formula I is from 0.05 to 10 millimol per 100 g of the hair dye.